Europäisches Patentamt

European Patent Office

Office européen des brevets

(19)

(11) Numéro de publication : **0 509 910 B1**

## (12) FASCICULE DE BREVET EUROPEEN

(45) Date de publication du fascicule du brevet :
06.07.94 Bulletin 94/27

(51) Int. Cl.$^5$ : **C22C 19/07, A61K 6/04**

(21) Numéro de dépôt : **92401049.9**

(22) Date de dépôt : **15.04.92**

(54) **Alliage à base de cobalt à faible coefficient de dilatation thermique.**

(30) Priorité : **19.04.91 FR 9104888**

(43) Date de publication de la demande :
**21.10.92 Bulletin 92/43**

(45) Mention de la délivrance du brevet :
**06.07.94 Bulletin 94/27**

(84) Etats contractants désignés :
**DE IT**

(56) Documents cités :
**EP-A- 0 041 938
EP-A- 0 195 351
DE-A- 2 147 991
DE-C- 3 436 118**

(73) Titulaire : **AUBERT & DUVAL
41, rue de Villiers
F-92200 Neuilly-Sur-Seine (FR)**

(72) Inventeur : **Frey, Jacques
18 avenue de Gouvieux
F-60260 Lamorlaye (FR)**

(74) Mandataire : **Dronne, Guy
Cabinet Beau de Loménie
158, rue de l'Université
F-75340 Paris Cédex 07 (FR)**

EP 0 509 910 B1

Jouve, 18, rue Saint-Denis, 75001 PARIS

**Description**

La présente invention concerne un alliage à base de cobalt à faible coefficient de dilatation thermique.

De façon plus précise, l'invention concerne un alliage à base de cobalt qui présente un faible coefficient de dilatation thermique, typiquement inférieur ou égal à 14,4.10$^{-6}$ m/m°C entre 20°C et 600°C, utilisable notamment pour la réalisation de prothèses dentaires. De préférence encore, l'alliage selon l'invention est plus particulièrement adapté à la réalisation de prothèses céramo-métalliques.

Comme on le sait, dans la réalisation de prothèses dentaires conjointes dites céramiques, une base métallique est recouverte de céramique de façon à donner, lorsque la prothèse est mise en place dans la bouche du patient, l'aspect d'une dent naturelle.

Les alliages les plus couramment utilisés pour les prothèses céramo-métalliques sont des alliages à base de nickel. Cependant, certaines observations faites ont conclu au caractère allergénique et même cancérigène du nickel de telle façon que les prothèses céramo-métalliques à base de nickel soit sont carrément interdites dans certains pays tels que la Suède, soit leur utilisation rencontre une vive réticence de la part de certains praticiens.

Pour remédier à ce problème, on commence à développer des alliages pour prothèse dentaire du type céramo-métallique à base de cobalt.

On comprend que pour la réalisation de telles prothèses, l'alliage doit présenter un certain nombre de caractéristiques parmi lesquelles on peut citer :
- les caractéristiques mécaniques qui dépendent de l'application ;
- la coulabilité pour donner à la chape métallique de la prothèse la forme convenable ;
- une aptitude au polissage ;
- une bonne résistance à la corrosion ;
- des propriétés de bio-compatibilité, et
- plus spécifiquement, des propriétés physiques compatibles avec le montage de la céramique sur la partie métallique.

La réalisation d'une prothèse céramo-métallique comporte les étapes principales suivantes :
- réalisation en cire perdue de l'élément prothétique ;
- préparation mécanique ou thermique de la surface métallique pour l'accrochage du matériau céramique ;
- dépôt d'un liant ou d'un lait opaque pour favoriser l'adhérence de la céramique suivi d'un opaque ; et
- dépôt de la céramique par couches successives avec des phases de cuisson intermédiaire.

On comprend que l'un des paramètres essentiels auquel doit satisfaire l'alliage utilisé pour permettre une bonne adhérence entre le métal et la céramique est que la céramique et l'alliage présentent des coefficients de dilatation thermique appariés. Plus précisément, le coefficient de dilatation thermique linéaire du métal doit être supérieur de 0,5 à 1.10$^{-6}$ m/m°C à celui de la céramique de façon à mettre celle-ci en compression lors du refroidissement de la prothèse après les phases de cuisson. Les céramiques utilisées habituellement pour réaliser les prothèses ont un coefficient de dilatation thermique voisin de 13,5.10$^{-6}$ m/m°C pour une plage de températures allant de 20° à 600°C.

De plus, les nouvelles céramiques utilisées et mises récemment sur le marché présentent un coefficient de dilatation thermique plus bas. En outre, les nouvelles tendances de la profession visent à augmenter la masse de céramique sur la chape métallique pour obtenir un meilleur rendu des teintes de la prothèse. Ces évolutions rendent encore plus nécessaire la mise au point d'alliages à coefficient de dilatation thermique plus bas pour être compatibles avec celui de la céramique.

On a déjà proposé des alliages pour prothèse dentaire céramo-métallique à base de cobalt qui présentent des coefficients de dilatation thermique le plus souvent supérieurs à 15.10$^{-6}$ m/m°C entre 20°C et 600°C. On comprend que ces alliages sont mal adaptés à la réalisation de prothèses céramo-métalliques du type mentionné ci-dessus.

Pour remédier à ces inconvénients un objet de l'invention est de fournir un alliage à base de cobalt qui présente un coefficient de dilatation thermique plus faible, tout en satisfaisant aux autres conditions énoncées ci-dessus.

Pour atteindre ce but, selon l'invention, l'alliage à base de cobalt à faible coefficient de dilatation thermique se caractérise en ce qu'il a la composition pondérale suivante :

| W+Mo | 10 à 15% | Si | 1 à 3% | B | 0 à 1% |
|------|----------|----|--------|---|--------|
| Cr | 22 à 27% | Mn | 0,3 à 0,8% | Fe < 2 % |
| C | 0,1 à 0,3% | Ni | < 1% | | |

le reste du poids étant constitué par du cobalt, et en ce que il présente un coefficient de dilatation thermique inférieur à $14,4.10^{-6}$.

Selon un premier mode préféré de mise en oeuvre de l'invention, l'alliage a la composition suivante :

| Mo | 10% | Si | 2% | Fe < 1% |
|----|-----|----|-----|---------|
| Cr | 25% | Mn | 0,6% | |
| C | 0,15% | Ni | < 0,6% | |

le cobalt constituant le reste du poids de l'alliage.

On obtient ainsi un alliage dont le coefficient de dilatation thermique est de $14,2.10^{-6}$ m/m°C entre 20°C et 600°C, ce qui permet de l'utiliser en particulier avec les céramiques dentaires présentant les plus bas coefficients de dilatation thermique.

Selon un deuxième mode préféré de mise en oeuvre, l'alliage a la composition pondérale suivante :

| Mo | 10% | Si | 2% | Co le reste |
|----|-----|----|-----|-------------|
| W | 5% | Mn | 0,6% | |
| Cr | 25% | Ni | < 0,6% | |
| C | 0,15% | Fe | < 1% | |

Un tel alliage présente un coeficient de dilatation thermique de $14,0.10^{-6}$ m/m°C entre 20°C et 600°C.

L'invention concerne également l'application de l'alliage défini ci-dessus à la réalisation de prothèses dentaires notamment céramo-métalliques.

D'autres caractéristiques et avantages de l'invention apparaîtront plus clairement à la lecture de la description qui suit de modes préférés de mise en oeuvre de l'invention donnés à titre d'exemples non limitatifs.

Comme on l'a déjà indiqué précédemment, selon l'invention, la composition pondérale de l'alliage est la suivante :

| W+Mo | 10 à 15% | Si | 1 à 3% | Fe < 2% |
|------|----------|----|--------|---------|
| Cr | 22 à 27% | Mn | 0,3 à 0,8% | B | 0 à 1% |
| C | 0,1 à 0,3% | Ni | < 1% | Co | le reste |

Il faut observer que dans la composition pondérale de l'alliage, il a été établi par le demandeur que la somme des pourcentages de tungstène et de molybdène doit être au moins égale à 10% pour obtenir les coefficients de dilatation thermique souhaités. En outre, ces deux métaux peuvent se substituer en partie au cobalt pour former avec celui-ci une solution solide. Outre ces deux métaux ajoutés au cobalt, il est nécessaire d'incorporer un certain pourcentage de chrome, au moins 22%, pour obtenir une bonne tenue de l'alliage à la corrosion, compte tenu des conditions d'utilisation dans la bouche du patient.

Il faut ajouter que, dans le cas où l'alliage comporte du carbone, dont la teneur est au maximum égale à 0,3%, le chrome, le molybdène et le tungstène s'associent au carbone pour former des carbures. Si la teneur en carbone est nulle ou très faible, ces éléments forment des phases intermétalliques avec le cobalt. L'alliage est donc constitué alors d'une solution solide et d'une ou plusieurs phases constituées par les carbures, ou une phase intermétallique.

Il est également possible d'ajouter du bore jusqu'à 1% du poids total pour améliorer encore la coulabilité de l'alliage lors de la réalisation de la chape métallique.

Les différents essais effectués ont permis de mettre au point une composition pondérale préférée qui est la suivante :

|         |       |    |       |    |          |
|---------|-------|----|-------|----|----------|
| Mo      | 10%   | Si | 2%    | Fe | < 1%     |
| Cr      | 25%   | Mn | 0,6%  | Co | le reste |
| C       | 0,15% | Ni | <0,6% |    |          |

Cette composition présente un coefficient de dilatation thermique de $14,2.10^{-6}$ m/m°C entre 20°C et 600°C.

En outre, cet alliage présente les caractéristiques suivantes :

Densité 8,3

Solidus 1250°C

Limite élastique (Rp 0,2): 700 MPa

Dureté Vickers (HV30) 380

Liquidus 1370°C

Résistance (Rm) 890 MPa

Allongement (A%) 4

On a réalisé des prothèses céramo-métalliques avec l'alliage défini ci-dessus dans les conditions opératoires suivantes :
- préparation du modèle de chape selon une forme du type "rateau" ;
- coulée de l'alliage en fronde à induction à l'air libre ;
- nettoyage de la chape ;
- montage d'une céramique "nouvelle génération" de coefficient de dilatation thermique $12,8.10^{-6}$ m/m°C, l'application de la masse céramique est précédée du dépôt d'une sous-couche constituée par un lait d'opaque très dilué suivi d'un opaque ; et
- cuissons successives de la céramique selon les spécifications du fabricant.

Cet essai a permis de faire les remarques suivantes :
- la coulabilité de l'alliage se révèle excellente ;
- le grattage et le polissage des parties métalliques aux différents stades de la fabrication sont très aisés ;
- l'ajustement entre chape métallique et empreinte est d'une très grande qualité grâce à la précision de coulée (grande finesse de détail) ;
- la prothèse céramo-métallique terminée, on ne constate aucune fissuration (dûe à l'alliage) dans la masse céramique, ni décollement à l'interface et ceci immédiatement après la pose ou beaucoup plus tard ;
- l'utilisation d'un lait d'opaque très dilué constitue d'ailleurs un test révélateur de la bonne compatibilité de l'alliage avec la céramique. Il s'agit en effet du mode de liaison métal-céramique le plus sensible à cette propriété et toute inadaptation du coefficient de dilatation d'un métal se traduit par des fissures dans la céramique.

Des essais comparatifs ont été faits avec un alliage à base cobalt de coefficient de dilatation thermique $15,3.10^{-6}$ m/m°C entre 20°C et 600°C, dans les conditions opératoires décrites précédemment.

On a observé des fissures de la masse céramique montrant l'incompatibilité due à la trop grande différence des coefficients de dilatation de l'alliage et de la céramique.

Avec l'alliage selon l'invention décrit ci-dessus, on a réalisé des prothèses céramo-métalliques dans les conditions suivantes :
- préparation d'un modèle suivant une forme type "sapin"
- coulée de l'alliage en fronde à induction ou au chalumeau
- après nettoyage, montage d'une céramique dite "à pâte crue", avec intermédiairement une sous-couche de "liant + opaque"
- cuissons de la céramique suivant protocole du fabricant de céramique.

Ce deuxième essai a permis de faire les observations suivantes :
- les opérations de grattage, polissage et la qualité de l'ajustement sont déclarées excellentes ainsi que la coulabilité ;
- l'oxydation, normale et naturelle, du métal que l'on constate habituellement lors des différentes opérations de coulée et de cuisson céramique, est très limitée. Son enlèvement est donc, de ce fait, facilité lors du travail de grattage et de finition pour les prothésistes. Par comparaison, on constate une oxydation moins forte que sur les alliages base nickel, classe Ni-Cr-B-Si, utilisés couramment pour la prothèse céramo-métallique.
- comme dans l'exemple 1, l'absence de fissuration est également constatée. De la même façon, un alliage base cobalt de coefficient de dilatation 15,3.10⁻⁶ m/m°C conduit dans les mêmes conditions de réalisation à des fissures dans la masse céramique.

Selon l'invention, on peut également utiliser un alliage ayant la composition pondérale suivante :

```
Mo  10%              Si   2%          Co le reste

W    5%              Mn  0,6%

Cr  25%              Ni < 0,6%

C  0,15%             Fe < 1%
```

Un tel alliage présente un coefficient de dilatation thermique égal à 14.10⁻⁶ m/m°C et une dureté Vickers (HV30) de 420.

En outre, l'aptitude de ces alliages et leur très bon comportement lors des opérations de coulée, de grattage, de polissage et d'ajustage rendent ceux-ci aptes à être utilisés pour la réalisation de prothèses conjointes simples (couronnes, bridges) non revêtues de céramiques.

## Revendications

1.  Alliage à base de cobalt à faible coefficient de dilatation, caractérisé en ce qu'il a la composition pondérale suivante :

    | | |
    |---|---|
    | Ensemble Molybdène + Tungstène | 10 à 15% |
    | Chrome | 22 à 27% |
    | Carbone | 0,1 à 0,3% |
    | Silicium | 1 à 3% |
    | Manganèse | 0,3 à 0,8% |
    | Bore | 0 à 1% |
    | Nickel | < 1% |
    | Fer | < 2% |
    | Cobalt | le reste du poids |

    et en ce qu'il présente un coefficient de dilatation thermique inférieur à 14,4.10⁻⁶ m/m°C entre 20°C et 600°C.

2.  Alliage selon la revendication 1, caractérisé en ce qu'il a la composition pondérale suivante :

    | | |
    |---|---|
    | Molybdène | 10% |
    | Chrome | 25% |
    | Carbone | 0,15% |
    | Silicium | 2% |
    | Manganèse | 0,6% |
    | Nickel | <0,6% |
    | Fer | <1% |
    | Cobalt | le reste du poids. |

3.  Alliage selon la revendication 1, caractérisé en ce qu'il a la composition pondérale suivante :

    | | |
    |---|---|
    | Molybdène | 10% |
    | Chrome | 25% |
    | Carbone | 0,15% |
    | Silicium | 2% |
    | Manganèse | 0,6% |
    | Nickel | <0,6% |
    | Fer | <1% |
    | Tungstène | 5% |
    | Cobalt | le reste du poids |

4.  Application de l'alliage selon l'une quelconque des revendications 1 à 3 à la réalisation de prothèses dentaires.

5. Application de l'alliage selon l'une quelconque des revendications 1 à 3 à la réalisation de prothèses dentaires céramo-métalliques.

## Patentansprüche

1. Legierung auf Kobaltbasis mit kleinem Ausdehnungskoeffizient,
dadurch gekennzeichnet,
daß sie die folgende Gewichtszusammensetzung hat:

| | |
|---|---|
| Molybdän + Wolfram zusammen | 10 bis 15% |
| Chrom | 22 bis 27% |
| Kohlenstoff | 0,1 bis 0,3% |
| Silizium | 1 bis 3% |
| Mangan | 0,3 bis 0,8% |
| Bor | 0 bis 1% |
| Nickel | < 1% |
| Eisen | < 2% |
| Kobalt | Rest des Gewichts |

und daß sie einen Wärmeausdehnungskoeffizient unter $14,4 \cdot 10^{-6}$ m/m °C zwischen 20 °C und 600 °C aufweist.

2. Legierung nach dem Anspruch 1, dadurch gekennzeichnet, daß sie die folgende Gewichtszusammensetzung hat:

| | |
|---|---|
| Molybdän | 10% |
| Chrom | 25% |
| Kohlenstoff | 0,15% |
| Silizium | 2% |
| Mangan | 0,6% |
| Nickel | < 0,6% |
| Eisen | < 1% |
| Kobalt | Rest des Gewichts. |

3. Legierung nach dem Anspruch 1, dadurch gekennzeichnet, daß sie die folgende Gewichtszusammensetzung hat:

| | |
|---|---|
| Molybdän | 10% |
| Chrom | 25% |
| Kohlenstoff | 0,15% |
| Silizium | 2% |
| Mangan | 0,6% |
| Nickel | < 0,6% |
| Eisen | < 1% |
| Wolfram | 5% |
| Kobalt | Rest des Gewichts. |

4. Verwendung der Legierung nach irgendeinem der Ansprüche 1 bis 3 zur Herstellung von Zahnprothesen.

5. Verwendung der Legierung nach irgendeinem der Ansprüche 1 bis 3, zur Herstellung von keramisch-metallischen Zahnprothesen.

## Claims

1. Alloy based on cobalt having a low coefficient of expansion, characterized in that its composition by weight is as follows:

| Molybdenum + Tungsten combination | 10 to 15% |
|---|---|
| Chromium | 22 to 27% |
| Carbon | 0.1 to 0.3% |
| Silicon | 1 to 3% |
| Manganese | 0.3 to 0.8% |
| Boron | 0 to 1% |
| Nickel | < 1% |
| Iron | < 2% |
| Cobalt | the remainder of the weight |

and in that its coefficient of thermal expansion is less that $14.6 \times 10^{-6}$ m/m°C between 20°C and 600°C.

2. Alloy according to claim 1, characterized in that its composition by weight is as follows:

| Molybdenum | 10% |
|---|---|
| Chromium | 25% |
| Carbon | 0.15% |
| Silicon | 2% |
| Manganese | 0.6% |
| Nickel | <0.6% |
| Iron | <1% |
| Cobalt | the remainder of the weight |

3. Alloy according to claim 1, characterized in that its composition by weight is as follows:

| Molybdenum | 10% |
|---|---|
| Chromium | 25% |
| Carbon | 0.15% |
| Silicon | 2% |
| Manganese | 0.6% |
| Nickel | <0.6% |
| Iron | <1% |
| Tungsten | 5% |
| Cobalt | the remainder of the weight |

4. Application of the alloy according to any one of claims 1 to 3 to the production of dentures.

5. Application of the alloy according to any one of claims 1 to 3 to the production of ceramo-metallic dentures.